# EUROPEAN PATENT APPLICATION

(11) **EP 3 632 410 A1**
(43) Date of publication of application: **08.04.2020**
(21) Application number: 18198987.2
(22) Date of filing: 05.10.2018
(51) Int. Cl.: A61K 9/00, A61K 9/127, A61K 48/00

(54) **LIPIDIC POLYNUCLEOTIDE CARRIERS FOR CELLULAR DELIVERY**

(71) Applicant: Lamellar Biomedical Limited, Eurocentral Holytown ML1 4WR (GB)
(72) Inventor: HOWARD, Lynsey, Holytown, ML1 4WR (GB); McLEAN, Alec, Holytown, ML1 4WR (GB)
(74) Representative: Murgitroyd & Company

(57) **Abstract**

A (phospho)lipid delivery composition for delivery of polynucleotide into cells.

## Description

### Field of the Invention

The invention provides a co-formulation of a (phospho)lipid composition (a combination of phospholipids and optionally cholesterol which form vesicles) and a polynucleotide wherein the co-formulation is capable of delivering the polynucleotide into a cell, the method of preparation of the co-formulation of the (phospho)lipid composition and polynucleotide and use of the same, for example to enhance the delivery of the polynucleotide into a cell. In particular, the present invention is directed to polynucleotide delivery and treatment of a disease or condition via delivery of polynucleotides to a cell. Further described is *in vitro* transfection and cell culture.

### Background

The introduction of biologically/pharmacologically active polynucleotide into disease sites and specific cells is required both in molecular biology and in relation to molecular medicine. Such biologically/pharmacologically active polynucleotides can comprise amongst others, DNA plasmids, RNA and RNA analogues or combinations thereof.

Over the last few decades, liposomes (lipid vesicle structures with specific lipid components), have been determined to be particularly effective in transferring biologically active compounds into cells and organs.

Liposomes have been found to be useful as delivery agents to introduce nucleic acids (e.g. DNA, RNA species), to cells. In particular, cationic liposomes have been shown to be effective for delivering anionic molecules to cells.

DNA liposome complexes, wherein liposomes contain phospholipids with lipids with amine end groups (the amines can be quaternary ammonium, tertiary, secondary or primary) which have positive surface charge (for example quaternary amines have a permanent positive charge at any pH), are considered as complexing negatively charged nucleotides and/or binding nucleotides on the inner and/or outer-surface of the phospholipid liposome vesicle membranes.

Such cationic liposomes, comprising cationic lipids, containing nucleic acids/nucleotides are known to be effective agents for efficient transfection of target cells. Liposomes can comprise concentrically arranged layers of phospholipids and may be categorised as small unilamellar vesicles, large unilamellar or multilamellar vesicles.

Several cationic lipids, for example DOTMA (N-[I-(2,3-dioleyloxyl) propyl]-N,N,N-trimethylammonium chloride), DOGS (5-carboxyspermylgly- cinedioctadecylamide) DOSPA (2,3-dioleyloxy-N-[2 (spermine-carboxamido) ethyl]-N,N-dimethyl-1-propanaminium), DC-Chol (N,N-dimethyl-N-ethylcarboxamidocholesterol), I ,4-bis(3-N-oleylamino-propyl)piperazine have been determined to be useful in delivery of nucleic acids/nucleotides to cells. Several reagents are commercially available. Some examples include LIPOFECTIN® (DOTMA:DOPE) (Invitrogen, Carlsbad, Calif.), LIPOFECTAMINE® (DOS-PA:DOPE) (Invitrogen), LIPOFECTAMINE® 2000 (Invitrogen) FUGENE®, TRANSFECTAM® (DOGS), EFFECT-ENEIRI, and DC-Chol. Liposomal carriers, characterised by the presence of amino-lipids carrying permanently a positive charge, irrespective of the pH of the environment (containing a quaternary nitrogen (N) atom) or a pH sensitive amino-lipids, being protonated at pH 4 and un-protonated at pH 7 (Tan, Y.Y.C. et al. Advances in Lipid Nanoparticles for siRNA delivery, Pharmaceutics 2013, 5, 498-507; doi:10.3390/pharmaceutics5030498), have been suggested to be toxic to cells, although the degree of toxicity varies from reagent to reagent. Further, it is considered such liposomes may be rapidly cleared by the body. The clearance has been reduced by suppressing receptor mediated uptake, endocytosis and phagocytosis of the liposomes using e.g. (optionally acid-labile) pegylated lipids, but problems with such methods still exist.

Lipoplexes, which are formed due to electrostatic interactions between negatively charged lipids, 1.2-dioleoyl-sn-phosphatidylethanolamine (DOPE) and negatively charged siRNA and divalent cations are also known in the art. Srinivasan, C and Burgess, D. (2009) Optimization and characterisation of anionic lipoplexes for gene delivery. Journal of Controlled Release 136: 62-70 discusses anionic lipoplexes comprising divalent cations, nucleic acids and anionic liposomes and the use of differing ratios of 1,2-dioleoyl-sn-phosphatidylglycerol (DOPG), (DOPE), various different divalent cations / nucleic acids (anions) ratios. Divalent cations are taught as being incorporated to achieve complexation between the RNA and the anionic lipids.

Membranes of different types of cells can restrict entry of extracellular material into cells. It is documented that the divalent cation containing lipid carriers do not always work efficiently (A. L. Barran-Berdonet al., Ca2+-Mediated Anionic Lipid - Plasmid DNA Lipoplexes. Electrochemical, Structural, and Biochemical Studies I Langmuir 2014, 30, 11704- 11713 dx.doi.org/10.1021/la502823z).

WO 01/72277 discusses (phospho)lipid vesicles and a method of preparing a polynucleic acid coding for an antigen within such (phospho)lipid vesicles, by hydration of a dry lipid blend with a saline medium containing solutes and a selected antigen wherein the dry lipid blend has been obtained from an organic solvent solution of the lipids by drying. This is described wherein the (phospho)lipid mixture (including cholesterol) in the percentages provided are dissolved in a chloroform / methanol solvent mixture (2:1 v/v) and the lipid solution is introduced into a round bottomed flask and attached to a rotary evaporator. The flask is evacuated and rotated at 60 r.p.m in a thermostatically controlled waterbath at a temperature of 30°C until a dry lipid film is deposited. Nitrogen is introduced into the flask and the residual solvent is removed before its connection to a lyophiliser where it is subjected to a high vacuum at room temperature for one hour. After release of the vacuum and following flushing with nitrogen, saline containing solutes (selected antigen) for entrapment is added. The lipid is hydrated within the flask, flushed with nitrogen, attached to the evaporator, and rotated at 60 r.p.m. at room temperature for thirty minutes. The suspension is allowed to stand for two hours at room temperature to complete the swelling process.

However, further work in this regard has determined that the method outlined in this teaching does not allow co-formulation of a nucleic acid, with a phospholipid delivery composition, for example wherein the nucleic acid is not encapsulated in a vesicle.

Teaching in the art suggests that RNA species should be encapsulated in carriers, since naked RNA (i.e. an aqueous solution of un-encapsulated RNA) is claimed to be not efficacious (Leung, AKK et al, Lipid Nanoparticles for Short Interfering RNA Delivery In: Advances in Genetics, Volume 88; Chapter 4, pp 72-102, 2014, Elsevier, Inc.).

In order to overcome the shortcomings of existing lipid carriers, for example to minimise possible toxicity of amino lipids (cationic lipids) containing carriers for gene transfer (including nucleic acids like DNA, RNA)/transfection, and / or enhance transfection efficiency, there exists a need for additional compositions that provide and suitably may enhance the delivery of a polynucleotide into a cell.

### Summary of the Invention

Accordingly, the present invention provides a co-formulated (phospho)lipid delivery composition and polynucleotide to allow intracellular delivery of the polynucleotide wherein the (phospho)lipid delivery composition does not contain amino lipids, pegylated lipids and divalent ions.

As used herein a (phospho)lipid delivery composition can be a combination of phospholipids and optionally cholesterol which form vesicles unless the composition is noted as explicitly excluding cholesterol.

In embodiments, the co-formulated (phospho)lipid delivery composition and polynucleotide to allow intracellular delivery of the polynucleotide consists essentially of the (phospho)lipid delivery composition and the polynucleotide to be delivered into a cell without any one or more of amino lipids, pegylated lipids and divalent ions.

In embodiments, the co-formulated (phospho)lipid delivery composition and polynucleotide to allow intracellular delivery of the polynucleotide consists of the (phospho)lipid delivery composition and the polynucleotide to be delivered into a cell without any one or more of amino lipids, pegylated lipids and divalent ions.

In embodiments, the co-formulated (phospho)lipid delivery composition and polynucleotide to allow intracellular delivery of the polynucleotide consists of the (phospho)lipid delivery composition and the polynucleotide to be delivered into a cell wherein the composition comprises negatively charged phospholipids at pH 7 and optionally cholesterol, but without amino lipids, pegylated lipids and divalent ions.

In embodiments the co-formulated (phospho)lipid delivery composition and polynucleotide, to allow intracellular delivery of the polynucleotide, can comprise a (phospho)lipid delivery composition, wherein the (phospho)lipid delivery composition does not comprise amino lipids or pegylated lipids, and the co-formulation will allow the phospholipid vesicles to deliver the polynucleotide in the absence of a further independent interaction moiety. Functionally, an interaction moiety allows interaction between the phospholipids and the polynucleotide, for example in the present invention, in embodiments no separate ions may be provided to aid interaction between the phospholipid and the polynucleotide, for example no divalent ions, or protein, or peptide or other polymer is provided within the lipid or at the lipid surface in addition to the phospholipid and polynucleotide of the co-formulation to allow the polynucleotide to be delivered into a cell.

Amino-lipids as discussed herein are amine or amino group containing lipids, being primary, secondary, tertiary or quaternary amines, where the quaternary amine lipids carry per definition a positive charge irrespective of pH. Other amine classes are pH sensitive, for example, the lipid is positively charged at pH values below the pKa (e.g. pH 4), and almost neutral at pH 7. The amino-lipids may be, for example, N,N-dioleyl-N,N-dimethylammonium chloride (DODAC), N,N-distearyl-N,N-dimethylammonium bromide (DDAB), N-(I-(2,3-dioleoyloxy)propyl)-N.N.N-trimethylammonium chloride (DOTAP), N-(I-(2,3-dioleyloxy)propyl)-N.N.N-trimethylammonium chloride (DOTMA), N,N-dimethyl-2,3-dioleyloxy)propylamine (DODMA), 1,2-DiLinoleyloxy-N,N-dimethylaminopropane (DLinDMA), 1,2-Dillinolenyloxy-N,Ndimethylaminopropane(DLenDMA), 1.2-Dilinoleylcarbamoyloxy-3-dimethylaminopropane (DLinC-DAP), 1,2-Dillinoleyoxy-3-(dimethylamino) acetoxypropane (DLin-DAC), 1,2-Dillinoleyoxy-3-morpholinopropane (DLin-MA), 1,2-Dillinoleoyl-3-dimethylaminopropane (DLinDAP), 1,2-Dillinoleylthio-3-dimethylaminopropane (DLin-S-DMA), 1-Linoleoyl-2- linoleyloxy-3-dimethylaminopropane (DLin-2-DMAP), (DLin-MC3-DMA) heptatriaconta-6,9,28,31-tetraen-19-yl 4-(dimethylamino)butanoate, 1.2-Dilinoleyloxy-3-trimethylaminopropane chloride salt (DLinTMAC1), 1,2-Dillinoleoyl-3-trimethylaminopropanechloride salt (DLin-TAPCI), 1,2-Dillinoleyloxy-3-(N-methylpiperazino)propane (DLin-MPZ), or 3-(N,N-Dillinoleylamino)-1,2-propanediol (DLinAP), 3-(N,N-Dioleylamino)-US 2016/0243255 A11,2-propanedio (DOAP), 1,2-Dillinoleyloxo-3-(2-N,Ndimethylamino) ethoxypropane (DLin-EG-DMA), 1.2-Dilinolenyloxy-N,N-dimethylaminopropane (DLinIMA), 2,2-Dillinoleyl-4-dimethylaminomethyl-1,3-dioxolane (DLin-K-DMA) or analogs thereof, (3aR.5s,6aS)-N,N-dimethyl-2,2-di((9Z,12Z)-octadeca-9,12-dienyl)tetrahydro3al-cyclopentad 1.3dioxol-5-amine (ALN100), (6Z.97,28Z.31Z)-heptatriaconta-6.9.28.31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (TechG1), or a mixture thereof.

Amino lipids derived from or which belong to the class of phospholipids (i.e. phosphorous containing lipids) of for instance phosphatidylethanolamine, phosphatidylserine and phosphatidylcholine; do not belong to amino lipids definition as used herein. Suitably, amino lipids derived from phosphatidylethanolamine, phosphatidylserine and phosphatidylcholine do not belong to amino lipids definition as used herein.

Pegylated lipids as discussed herein are conjugated lipids with polyethyleneglycol (polyethyleneglycol (PEG)-lipid) including, without limitation, a PEG-diacylglycerol (DAG), a PEG-dialkyloxypropyl (DAA), a PEG-phospholipid, a PEG-ceramide (Cer), Cholesterol-PEG derivatives or a mixture thereof. They may be sensitive or less sensitive to intracellular hydrolysis, dependent on their chemical structure. The PEG DAA conjugate may be, for example, a PEG-dilauryloxypropyl (Ci), a PEG-dimyristyloxypropyl (Cia), a PEGdipalmityloxypropyl (Ci), or a PEG-distearyloxypropyl. (Cls) or a mixture thereof. Other examples of PEG conjugates include PEG-cDMA (N-[(methoxy polyethylene glycol)2000) carbamyl]-dimyristyloxlpropyl-3-amine), mPEG2000-DMG (mPEG-dimyrystylglycerol (with an average molecular weight of 2000 D), (polyethylene glycol-dimyristolglycerol, PEG-DMG) and PEG-C-DOMG (R-3-[(ω-methoxy-poly(ethyleneglycol)2000)carbamoyl)]-1,2-dimyristyloxlproppyl-3-amime).

Suitably, in embodiments a (phospho)lipid delivery composition may comprise a lipid part of the (phospho)lipid delivery composition comprising at least one of the lipids selected from PC, PE, PS, PI, PG, PA, and sphingomyelin (SM) and optionally cholesterol wherein the (phospho)lipid delivery composition must comprise a negatively charged phospholipid at pH 7 optionally selected from at least one of PI, PS, PG and PA.

In embodiments a co-formulated (phospho)lipid delivery composition and polynucleotide can be wherein the polynucleotide is provided
1) simultaneously as
   i) a mixture of delivery composition and non-encapsulated/non-associated polynucleotide, or
   ii) a mixture of delivery composition with non-encapsulated/non-associated polynucleotide in combination with an encapsulated/associated polynucleotide
      or,
2)
   a) sequentially, as a separate delivery composition and a separate solution of the polynucleotide which can be provided in combination to a subject or a cell to provide a co-formulated delivery composition without a further active substance or
   b) sequentially, as a separate delivery composition and a separate solution of the polynucleotide which can be provided in combination to a subject or a cell to provide a co-formulated delivery composition with a further encapsulated/associated active substance wherein the active substance is not an independent interaction moiety.

By associated it can be considered the polynucleotide is bound to the outside of vesicles provided by the (phospho)lipid delivery composition, for example tethered by ionic binding.

Without wishing to be bound by theory, it is considered the co-formulated (phospho)lipid delivery composition provides a cell penetrating function and is capable of delivering the polynucleotide to and into a cell or cells of an organ without requiring encapsulation of the polynucleotide by the (phospho)lipid delivery composition. Advantageously the process to provide the (phospho)lipid delivery composition and polynucleotide co-formulation does not require active encapsulation steps.

Additionally, an active substance, for example a drug product, intended to furnish pharmacological activity or other direct effect in the diagnosis, cure, mitigation, treatment, or prevention of disease, or to affect the structure or any function to a cell or body of a human or other animal may be provided with the (phospho)lipid delivery composition. An active substance can include a substance that may undergo chemical change during the manufacture or delivery of the substance, for example a pro-drug product and be present or be provided to a cell in a modified form to furnish a specified activity or effect. Suitably an active substance may be a tag or substrate to allow visualisation of the (phospho)lipid delivery composition and / or the polynucleotide being delivered by the (phospho)lipid composition in co-formulation. In such embodiments, a tag or substrate may provide luminescence to indicate a condition, disease, genetic disorder or infection. This may provide for improved diagnostic techniques in addition to delivery of a polynucleotide. Suitably the polynucleotide may be a probe nucleic acid which allows identification of a gene or nucleic acid sequence in a cell. Suitably the probe may allow the detection of expression or non-expression of a nucleic acid sequence by the cell. Suitably the probe may allow the detection of whether a nucleic acid sequence in a cell is accessible to binding or the location of a nucleic acid sequence within a cell.

Advantageously, the co-formulated (phospho)lipid delivery composition and polynucleotide may be provided with an active substance which may optimize the cellular uptake and intracellular trafficking of the polynucleotide to be delivered to the cell, may provide for lower systemic or local toxicity and / or may enable improved delivery of a polynucleotide to a target cell than conventional means, for example wherein a target cell is covered with a layer of viscous mucus, the (phospho)lipid composition may allow improved delivery of polynucleotide and a further active substance may offer a superior local or systemic therapy of diseases like e.g. cystic fibrosis and other genetic disorders.

Advantageously, the co-formulated (phospho)lipid delivery composition and polynucleotide, may be useful in improving transfection efficiency and / or decreasing toxicity or phenotypic effects to cells to which the polynucleotide is delivered, i.e. to transfected cells. The toxicity which may be minimised may be local or systemic toxicity, for example inflammatory response which can be observed when cationic liposomes are provided to cells.

Unexpectedly, despite the negative charge provided at the surface of the (phospho)lipid delivery composition of the present invention, it is able to provide polynucleotide / nucleic acid entry into cells. This is atypical, for example a review on pulmonary delivery on siRNA is silent on the topical use of lipid carriers containing negatively charged lipids (Ka-Wing Lam J et al., Pulmonary delivery of therapeutic siRNA, Advanced Drug Delivery Reviews 64 (2012) 1-15). The review mentions the use of naked RNA, but not in simultaneous combination with or sequential administration of lipid carriers.

Further, unlike as described for lipoplexes, formed due to electrostatic interactions between positively charged amino lipids and negatively charged RNA or negatively charged lipids and negatively charged RNA via divalent cations, it is considered that amino lipids and pegylated lipid and divalent ions are not required to provide the co-formulated (phospho)lipid delivery composition and the polynucleotide to allow intracellular delivery of the polynucleotide.

The (phospho)lipid delivery composition for co-formulation with polynucleotide to allow cellular delivery of the polynucleotide allows the introduction of the polynucleotide into and to cells in culture or in cells in tissue *in vivo*. Suitably it may be used in gene transfer for local gene therapy to treat any disease with a genetic component or where transfected genetic material (for example a nucleic acid) may constitute an appropriate treatment approach e.g. idiopathic pulmonary fibrosis, fibrotic lung diseases, or other lung diseases, including those characterised by increased mucous secretion and diseases and/or conditions which alter mucous secretions, for example cystic fibrosis and COPD.

In embodiments, a (phospho)lipid delivery composition of the co-formulated (phospho)lipid delivery composition and polynucleotide can comprise a lamellar body lipid composition and polynucleotide to be delivered to the interior of a cell.

In embodiments a co-formulated (phospho)lipid delivery composition with a polynucleotide represents aqueous liquid dispersions of phospholipid vesicles. The vesicles may comprise water soluble genetic material (nucleic acid). Alternatively, vesicles may be arranged around a polynucleotide to effectively sheath or cloak a polynucleotide. Suitably any other water soluble or lipophilic compound of therapeutic interest, or combinations thereof may also be provided. Suitably the lipid composition of the (phospho)lipid delivery compositions reflects the lipid composition of lamellar bodies or contains lipids which are anionic at pH 7, (including phosphatidylglycerol, phosphatidylserine, phosphatidic acid or phosphatidylinositol) to efficiently deliver the genetic material (nucleic acid) and any other water soluble or lipophilic compound of a therapeutic of interest to a cell and/or disease site. The lipids may form a lamellar membrane surrounding at least one aqueous compartment. The (phospho)lipid delivery composition differs from natural lamellar bodies in that it does not contain endogenous proteins, which occur in naturally occurring lamellar bodies (Schmitz G and Muller G. Structure and function of lamellar bodies, lipid-protein complexes involved in storage and secretion of cellular lipids J Lipid Res.1991 Oct;32(10):1539-70).

The (phospho)lipid delivery composition may also be described as a lipid imitation of an exosome. Exosomes are a natural transport system carrying biological information, including RNA (Babae, N., et al., Systemic miRNA-7 delivery inhibits tumor angiogenesis and growth in murine xenograft glioblastoma, Oncotarget., 2014, 5(16), 6687-700.; Van der Meel, R., et al, Extracellular vesicles as drug delivery systems: lessons from the liposome field, J. Control. Release, 2014, 195, 72-85.; Zomer, A., et al., In Vivo imaging reveals extracellular vesicle-mediated phenocopying of metastatic behavior. Cell., 2015, 161(5), 1046-1057.), which is exchanged between cells. This transport system consists of particles surrounded by a (phospho)lipid membrane. Several exosome populations have been recognized based on their intracellular origin and include exosomes and microvesicles (Colombo, M, Raposo, G., Théry, C., Biogenesis, secretion, and intercellular interactions of exosomes and other extracellular vesicles, Annu. Rev. Cell. Dev. Biol., 2014, 30, 255-89.). Exosomes originate from multivesicular bodies, i.e. endosomes that contain intraluminal vesicles. When multivesicular bodies fuse with the plasma membrane, these intraluminal vesicles are secreted, and subsequently referred to as exosomes. Exosomes comprise a selected subset of lipids (including e.g. sphingomyelin, phosphatidylcholine, phosphatidylethanolamine, phosphatidylserine, ganglioside GM3, and phosphatidylinositol, which may be tissue specific (Skotland, T., Sandvig, K., Llorente, A., Lipids in exosomes: Current knowledge and the way forward; Progress in Lipid Research, 2017, 66, 30-41.)

The (phospho)lipid delivery composition may be described as "carrier" or "lipid carrier", which represents a vesicle or liposome like structure comprising one or more lipid lamellar membranes surrounding one or more aqueous compartments.

The (phospho)lipid delivery composition may be provided in a sterile manner by applying for example sterile filtration through a 0.2 µm pore size filter or alternative methods known in the art.

Suitably, the (phospho)lipid delivery composition suitable for the delivery of a polynucleotide to the interior of a cell as part of a co-formulation, may be used for *in-vitro* and *in-vivo* transfection of cells. In particular, the present invention may advantageously provide compositions that enhance the transfection efficiency of cells, including those cell types that are considered to typically be difficult to transfect and / or show reduced toxicity to cells when compared to cationic liposomes. Suitably a co-formulated (phospho)lipid delivery composition as disclosed herein may increase the delivery of polynucleotide, suitably increase the transfection efficiency of a polynucleotide, for example a DNA or RNA nucleic acid by up to 25%, up to 30%, up to 35%, up to 40%, up to 45%, up to 50%, up to 55%, up to 60%, up to 65%, up to 70%, up to 75%, up to 80%, up to 85%, up to 90%, up to 95%, up to 100% or in excess of 100% over a typical cationic liposome to which the polynucleotide is provided.

Suitably, the co-formulated (phospho)lipid delivery composition and polynucleotide may be a (phospho)lipid delivery composition comprising as a polynucleotide at least one of miR-155, miR-29, or miR-212. Each of these miR (microRNA and miRNA) are known in the art.

Suitably, in addition to a polynucleotide, a water soluble drug substance may be present inside and / or outside the (phospho)lipid delivery composition (lipid carrier).

Suitably the polynucleotide, for example RNA or DNA nucleic acid may be only outside (non-encapsulated) the vesicles provided by the (phospho)lipid delivery composition and may not be bound to the outside of the vesicles, for example not tethered by ionic binding.

Suitably an active substance provided in addition to the polynucleotide may be substantially (i.e. more than 90 % of total) only outside a vesicle provided by the (phospho)lipid delivery composition.

Suitably, the (phospho)lipid delivery composition may comprise a lipophilic drug or other active substance contained within the lamellar membrane of the vesicle of the composition. Suitably a water soluble active substance or water soluble compound may be encapsulated by the vesicle of the co-formulated (phospho)lipid delivery composition.

Suitably, the co-formulated (phospho)lipid delivery composition with a polynucleotide, to allow intracellular delivery of the polynucleotide, may comprise a weight ratio of polynucleotide to total lipid from 1:1000 to 10:1.

Suitably, the co-formulated (phospho)lipid delivery composition with a polynucleotide, to allow intracellular delivery of the polynucleotide, may comprise a weight ratio of polynucleotide to total lipid from 1:100 to 1:1.

Suitably, the co-formulated (phospho)lipid delivery composition with a polynucleotide, to allow intracellular delivery of the polynucleotide, may comprise a weight ration of polynucleotide to total lipid from 1:10 to 1:50.

Suitably, the co-formulated (phospho)lipid delivery composition with a polynucleotide, to allow intracellular delivery of the polynucleotide, may comprise a weight ratio of polynucleotide to total lipid between 1:1 and 1:1000.

Suitably, the co-formulated (phospho)lipid delivery composition with polynucleotide to allow intracellular delivery of the polynucleotide may comprise a weight ratio of polynucleotide to total lipid in the range 10:1 to 20:1.

### Lipid composition

Suitably the delivery of the polynucleotide to the cell may be provided without inducing an inflammatory response or inducing an inflammatory response less than typically obtained following administration of cationic (amino-lipid) particles by a cell. Suitably, the (phospho)lipid delivery composition for co-formulation with polynucleotide to allow intracellular delivery of the polynucleotide may be provided such that it can be administered to an animal or to a human patient that would benefit from the administration thereof for the delivery of the polynucleotide to the interior of a cell of such a patient. Suitably, the (phospho)lipid delivery composition may contain at least one phospholipid, which is negatively charged at pH 7. Advantageously, in embodiments the (phospho)lipid delivery composition always at least one phospholipid, which is negatively charged at pH 7.

Suitably, the (phospho)lipid delivery composition for co-formulation with a polynucleotide and optionally with another active substance to allow intracellular delivery of the polynucleotide and optionally another active substance may be provided without the need for ions in combination with negatively charged lipids of the (phospho)lipid delivery composition, for example without the need for divalent ions such as calcium, and without the need for amino-lipids to be provided to allow the interaction of a (phospho)lipid delivery composition with the polynucleotide, for example a DNA or RNA nucleic acid sequence.

The ability to form a co-formulated (phospho)lipid delivery composition with a polynucleotide to allow intracellular delivery of the polynucleotide in the absence of divalent ions is in contrast to anionic phospholipid vesicles which require, besides DOPE, a divalent ion, typically e.g. Ca²⁺ or Mg²⁺ or Zn²⁺, to act as a bridge to negate repulsion from a nucleic acid molecule and allow lipoplex formation.

The ability to form a co-formulated (phospho)lipid delivery composition and polynucleotide to allow intracellular delivery of the polynucleotide in the absence of amino lipids is in contrast with the use of so-called Lipid Nano Particles, which comprise pH-sensitive amino lipids and (acid-labile) peg-ylated lipids (Tan, Y.Y.C. et al. Advances in Lipid Nanoparticles for siRNA delivery, Pharmaceutics 2013, 5, 498-507; doi:10.3390/pharmaceutics5030498).

In contrast to natural lamellar bodies, the (phospho)lipid delivery compositions do not require to contain endogenous proteins related to physiologically occurring lamellar bodies.

As discussed herein, in addition to delivery of a polynucleotide, the (phospho)lipid delivery composition may additionally allow delivery of lipophilic compounds for example lipophilic active substances or water soluble compounds for example water soluble active substances.

In embodiments the co-formulated (phospho)lipid delivery composition and polynucleotide do not contain pegylated (phospho)lipids, which may be acid labile.

Suitably a co-formulated (phospho)lipid delivery composition and polynucleotide to allow intracellular delivery of the polynucleotide may comprise at least one of the components selected from the phospholipids: phosphatidylcholine, phosphatidylglycerol, suitably for example as a sodium or ammonium salt, phosphatidic acid, suitably for example as sodium or ammonium salt, sphingomyelin, phosphatidylethanolamine, phosphatidylserine, suitably for example as a sodium salt, phosphatidylinositol, suitably for example as a sodium salt and, in combination with at least one of aforementioned phospholipids, cholesterol.

The (phospho)lipid delivery composition may comprise phosphatidylglycerol as lipid, which may be of advantageous with respect to compatibility and tolerability, when the compositions will be administered to the lung wherein phosphatidylglycerol is an endogenous lipid.

Suitably the (phospho)lipid delivery composition may comprise a phospholipid and a lipid that provides vesicles of the delivery composition with an anionic charge.

Suitably the (phospho)lipid delivery composition may comprise phosphatidyl choline and a negatively charged lipid (at pH 7). Optionally a negatively charged lipid may be selected from at least one of phosphatidyl inositol, phosphatidylserine, phosphatidic acid, or phosphatidylglycerol. Suitably, the (phospho)lipid delivery composition may comprise phosphatidyl choline and sphingomyelin and a negatively charged lipid (at pH 7). Optionally a negatively charged lipid may be selected from at least one of phosphatidyl inositol, phosphatidylserine, phosphatidic acid, or phosphatidylglycerol. Suitably the (phospho)lipid delivery composition may comprise phosphatidylcholine and cholesterol and a negatively charged lipid (at pH 7). Optionally a negatively charged lipid may be selected from at least one of phosphatidyl inositol, phosphatidylserine, phosphatidic acid, or phosphatidylglycerol.

Suitably, the (phospho)lipid delivery composition may comprise a combination of cholesterol, sphingomyelin (SM), phosphatidylcholine (PC), phosphatidylethanolamine (PE), phosphatidylserine (PS), phosphatidic acid (PA), glycolipids and phosphatidylinositol (PI) and phosphatidylglycerol (PG). Suitably phosphatidylglycerol may be used in greater proportions to replace PI or PS or to replace both of PS and PI. Suitably PA may be provided to replace PI or PS or to replace both of PS or PI as appropriate. Suitably the glycolipid may be provided at 0 - 25% by weight.

Suitably the co-formulated (phospho)lipid may comprise or consist of cholesterol, sphingomyelin, phosphatidylcholine and a negatively charged lipid (at pH7), for example wherein the negatively charged lipid is optionally phosphatidyl inositol, phosphatidylserine, phosphatidic acid, or phosphatidylglycerol.

Suitably in use, the lipids may be provided in ranges of: - 20-80% phosphatidylcholine may be provided, about 0-23% sphingomyelin may be provided, about 0-10% phosphatidylethanolamine may be provided, about 0-6% phosphatidylserine may be provided, about 0-4% phosphatidylinositol may be provided, about 0-12% cholesterol may be provided, about 0-25% phosphatidylglycerol may be provided, about 0-25% phosphatidic acid may be provided and about 0-25% glycolipid may be provided, in combinations as discussed herein to provide a delivery composition of the present invention.

In embodiments the co-formulated (phospho)lipid delivery composition and polynucleotide to allow intracellular delivery of the polynucleotide can comprise or consist essentially of these constituents in the range of about, in relation to total lipid content, 20-80 % phosphatidylcholine, about 0-23% sphingomyelin, about 0-10% phosphatidylethanolamine, about 0-6% phosphatidylserine, about 0-4% phosphatidylinositol and about 0-12% cholesterol about 0-25 % phosphatidylglycerol and about 0-25% phosphatidic acid by weight.

Suitably, the co-formulated (phospho)lipid delivery composition and polynucleotide to allow intracellular delivery of the polynucleotide may further comprise about 0-3% by weight lysophosphatidylcholine, either as separately added component or as impurity of the used phosphatidylcholine.

Suitably, the co-formulated (phospho)lipid delivery composition and polynucleotide may comprise, in relation to total lipid content, about 54% phosphatidylcholine, about 19% sphingomyelin, about 8% phosphatidylethanolamine, about 4% phosphatidylserine, about 3% phosphatidylglycerol and about 10% cholesterol by weight. In another embodiment the preferred composition of the co-formulated (phospho)lipid delivery composition may further comprise about 2% lysophosphatidyl choline by weight.

Suitably, the co-formulated (phospho)lipid delivery composition and polynucleotide to allow intracellular delivery of the polynucleotide may comprise, in relation to total lipid content, phosphatidylcholine 44-80%, sphingomyelin 0-25%, phosphatidylethanolamine 0-25%, phosphatidylserine 0-10%, phosphatidylinositol 0-4% and cholesterol 0 -50%.

Suitably, the co-formulated (phospho)lipid delivery composition and polynucleotide may comprise related to total lipid content, phosphatidylcholine 44-100%, sphingomyelin 0-25%, phosphatidylethanolamine 0-10%, phosphatidylglycerol 0-20% and cholesterol 0-50%.

Suitably a (phospho)lipid, which is negatively charged at pH 7, may be selected from the group consisting of phosphatidylserine, phosphatidylinositol, phosphatidic acid and phosphatidylglycerol.

It is understood that further lipid components of the co-formulated (phospho)lipid delivery composition may be impurities in the used lipids generated during production or storage.

Suitably, the co-formulated (phospho)lipid delivery composition and polynucleotide may comprise phosphatidylcholine, sphingomyelin, phosphatidylethanolamine, phosphatidylglycerol and cholesterol.

Suitably the phospholipids of the (phospho)lipid delivery composition may be synthetic, semi synthetic or natural (derived from soybean, sunflower or egg yolk). They may contain esterified mono or poly saturated or unsaturated fatty acids. Suitably, the co-formulated (phospho)lipid delivery composition and polynucleotide may comprise sphingomyelin, phosphatidylcholine, phosphatidylethanolamine, and phosphatidylserine.

Suitably, the co-formulated (phospho)lipid delivery composition and polynucleotide to allow intracellular delivery of the polynucleotide may comprise phosphatidylcholine, phosphatidylethanolamine, and phosphatidylserine.

Suitably, the co-formulated (phospho)lipid delivery composition and polynucleotide may comprise an additional (phospho)lipid component in present in natural phospholipids from e.g. egg or soybean containing mainly phosphatidylcholine

Suitably lyso PC, lyso PS, and lyso PE may be added as separate components or included as additional component in natural phospholipids.

Suitably phosphatidylethanolamine may be an additional component in natural phospholipids form e.g. egg or soybean containing mainly phosphatidylcholine Suitably the sphingomyelin component is Egg SM.

Suitably, the PS component may be selected from the group consisting of DOPS (1,2 dioleoylphosphatidylserine) or DMPS (1,2 dimyristoylphosphatidylserine) or Soy PS in sodium salt form.

Suitably, the PG component may be selected from the group consisting of DPPG, DMPG (1,2 dimyristoylphosphatidylglycerol), DSPG (1,2-Distearoyl-sn-glycero-3-phosphoglycerol), POPG (1-palmitoyl, 2-oleoylphosphatidylglycerol), Egg PG in sodium or ammonium salt form.

Suitably, the PC component may be selected from the group consisting of Soybean PC, containing more than 65 % phosphatidylcholine, Egg PC, containing more than 65 % PC, DOPC (1,2 dioleoylphosphatidylcholine), POPC (1-palmitoyl, 2-oleoylphosphatidlycholine), DMPC (1,2 dimyristoylphosphatidylcholine), DEPC (1,2 di-erucoylphosphatidylcholine).

Suitably, the PC component may be selected from the group consisting of Soybean PC, containing more than 95 % phosphatidylcholine, Egg PC containing more than 95 % PC.

Suitably, the PE component may be selected from DOPE (1,2 dioleoylphosphatidylethanolamine), POPE (1-palmitoyl, 2-oleoyl phosphatidylethanolamine) or Egg PE.

Suitably, the co-formulated (phospho)lipid delivery composition and polynucleotide may comprise sphingomyelin, phosphatidylcholine, phosphatidylethanolamine and phosphatidylglycerol and be provided in a lamellar body like structure.

Suitably, the co-formulated (phospho)lipid delivery composition and polynucleotide may comprise cholesterol, sphingomyelin, phosphatidylcholine, phosphatidylethanolamine and phosphatidylglycerol and be provided in a lamellar body like structure.

Suitably, the co-formulated (phospho)lipid delivery composition and polynucleotide may comprise phosphatidylcholine, phosphatidylethanolamine and phosphatidylglycerol and be provided in a lamellar body like structure.

Suitably, the co-formulated (phospho)lipid delivery composition and polynucleotide may comprise cholesterol, phosphatidylcholine, phosphatidylethanolamine and phosphatidylglycerol and be provided in a lamellar body like structure.

Suitably, the co-formulated (phospho)lipid delivery composition and polynucleotide may comprise phosphatidylcholine and phosphatidylglycerol and be provided in a lamellar body like structure.

Suitably, the co-formulated (phospho)lipid delivery composition and polynucleotide may comprise cholesterol, phosphatidylcholine and phosphatidylglycerol and be provided in a lamellar body like structure. Optionally additionally lipid components may be provided, for example phosphatidic acid and / or sphingomyelin.

Suitably, the co-formulated (phospho)lipid delivery composition and polynucleotide may comprise cholesterol, phosphatidylcholine and phosphatidic acid and be provided in a lamellar body like structure. Optionally the co-formulated (phospho)lipid may comprise sphingomyelin.

Suitably, in addition to polynucleotide, a further active substance may be provided.

### Morphology and size

In certain embodiments the (phospho)lipid delivery composition can be a lamellar body like structure comprising unilamellar, multilamellar, multivesicular, or bilayered phospholipid structures. Suitably vesicles provided by the (phospho)lipid delivery composition may be small unilamellar vesicles, large unilamellar or multilamellar vesicles. Lamellar bodies form self-closed structures which can be of varying size with a hydrophilic core. The co-formulated (phospho)lipid delivery composition may adopt a substantially spherical structure ranging from around 20 nm, 50 nm, suitably 80 nm to 200 nm diameter. Optionally, they may, however, be larger than 200 nm in diameter, even up to 20 µm, or even 80 µm in diameter. At 20µm or 80µm measurements may not be of single, discrete vesicles but aggregates. The (phospho)lipid delivery composition may be very heterogeneous or very homogenous with respect to particle size distribution. In embodiments the phospholipids may adopt a structure with of around 0.15 µm, suitably up to 0.20 µm diameter with a zeta potential ranging from about -4 mV to about -70 mV. Suitably a zeta potential may range from about -15 mV to about -40 mV. It is considered that stability of the vesicles will be assessed in relation to physical stability - the ability to retain a defined vesicle size in the ranges provided and / or chemical stability - the ability to resist degradation, for example with suitable zeta potential to cause repulsion between two vesicles. Stability may be assessed for stability over a time frame of at least 6 months, at least 9 months, at least 12 months, at least 24 months, at least 36 months. Suitably stability may be assessed at a temperature in the range 2 to 8°C. Suitably structures may be stable in aqueous dispersion in the range about pH 3 and about pH 12, suitably between pH 6 to 8, in particular in the range 6.5 to 7.0.

To assess zeta potential, an instrument (e.g. Zetasizerfrom Malvern Panalytical Limited) can be used to apply an electric field and a direction and velocity of the motion of the vesicles can be related back to the charge of the vesicle. The value is influenced by the suspending medium and instrument settings and thus standardized conditions are applied.

Suitably the (phospho)lipid delivery composition may comprise phospholipids which are negatively charged at pH 7.

Suitably the (phospho)lipid delivery composition when dispersed in an aqueous medium, may have an average size of more than 200 nm.

Suitably the (phospho)lipid delivery composition when dispersed in an aqueous medium, may have an average size of 200 nm or smaller.

Suitably the co-formulated (phospho)lipid delivery composition and polynucleotide may comprise only structures with one phospholipid lamellar membrane (unilamellar). Suitably the co-formulated (phospho)lipid delivery composition and polynucleotide may comprise only structures with at least two lamellar membranes (multilamellar).

In embodiments where the polynucleotide is a nucleic acid capable of regulating expression, for example a miRNA, the co-formulated (phospho)lipid delivery composition and polynucleotide may be contacted with a cell such that the nucleic acid, for example a miRNA is transported into the cell where it may allow upregulation, downregulation or expression of a target gene product or target gene products.

Where a nucleic acid molecule is provided to a cell to inhibit expression of a gene product, expression of a gene product can be inhibited by at least 5%, at least 10%, at least 15%, at least 20%, at least 25%, at least 30%, at least 50%, at least 75%, at least 90%, at least 95%, at least 99% or 100% compared to expression of the gene product in the absence of the (phospho)lipid delivery composition. Where the nucleic acid molecule is provided to allow expression or upregulation of a gene product, expression of a gene product can be provided or increased by at least 5%, at least 10%, at least 15%, at least 20%, at least 25%, at least 30%, at least 50%, at least 75%, at least 90%, at least 95%, at least 99% at least 100% or by more than 100% compared to expression of the gene product in the absence of the (phospho)lipid delivery composition. Several factors may influence the effect of nucleic acid provided to a cell including but not limited to dosage/concentration of the biologically active molecule, biological redundancy, biologically active molecule design (for example siRNA sequence), cell type, cell line, epigenetics, proteomics or genomics.

According to a second aspect of the invention there is provided a method of preparing a co-formulated (phospho)lipid delivery composition and polynucleotide to allow intracellular delivery of the polynucleotide wherein the (phospho)lipid delivery composition does not contain any one or a combination of amino lipids, pegylated lipids and divalent ions comprising the step of mixing the (phospho)lipid delivery composition with a polynucleotide without the need to further provide amino lipids, pegylated lipids or divalent ions.

Suitably the (phospho)lipid delivery composition may be manufactured by mixing a pre-prepared liposome, made by any method known in the art, and an aqueous solution of the nucleotide as the desired lipid to nucleotide ratio

According to a third aspect of the present invention there is provided the use of the co-formulated (phospho)lipid delivery composition and polynucleotide of the first aspect in medicine.

According to a further aspect of the present invention there is provided the use of co-formulated (phospho)lipid delivery composition and polynucleotide of the first aspect in a method of therapy.

According to a further aspect of the present invention there is provided a co-formulated (phospho)lipid delivery composition and polynucleotide of the first aspect for treatment of a disease or condition.

According to a further aspect of the present invention there is provided a method of administering a therapeutically effective amount of polynucleotide co-formulated with a (phospho)lipid delivery composition of the first aspect to a subject in need thereof.

Suitably, in addition to the polynucleotide, a further active substance may be provided to the (phospho)lipid delivery composition, wherein the further active substance is provided in the phospholipid layers of the vesicles of the (phospho)lipid delivery composition or encapsulated within the vesicles.

Suitably the co-formulated (phospho)lipid delivery composition may be advantageously used as a topical medication for treatment of diseases characterised by increased secretion of viscous mucus. This would allow the (phospho)lipid composition to combine its mucus diluting property and drug delivery properties. Suitably the (phospho)lipid delivery composition may be used in combination with other viscous mucus diluting medications. The co-formulated (phospho)lipid delivery composition and polynucleotide may be particularly suitable for treatment of diseases which are characterised by an increased secretion of viscous mucus, biofilms or biological matrices. For example, the (phospho)lipid delivery composition may enhance the ability of topically applied medications in reaching diseased cells.

Suitably the co-formulated (phospho)lipid delivery composition and polynucleotide may be suitable for treatment of genetic disorders.

Suitably the co-formulated (phospho)lipid delivery composition and polynucleotide may be advantageously used for treatment of bronchitis, asthma and cystic fibrosis.

Suitably the co-formulated (phospho)lipid delivery composition and polynucleotide may be advantageously used for treatment of idiopathic pulmonary fibrosis and other interstitial lung diseases.

Suitably the co-formulated (phospho)lipid delivery composition and polynucleotide may be used for in-vivo or in-vitro diagnostics.

Suitably the co-formulated (phospho)lipid delivery composition and polynucleotide may be used for ex-vivo manipulation of cells isolated from the body, followed by re-administration of the manipulated cells. For example, the co-formulated composition may allow delivery of nucleic acid to dendritic cells to modulate immune responses,

Suitably the co-formulated (phospho)lipid delivery composition and polynucleotide may be suitable for treatment of diseases, which benefit from the interaction mechanisms of the (phospho)lipid delivery composition with the diseased cells or organs.

Suitably the co-formulated (phospho)lipid delivery composition and polynucleotide may be used for delivery to cells by means of mechanisms selected from the group consisting of endocytosis, pinocytosis, macro-pinocytosis and phagocytosis.

### Use and Administration

Suitably the (phospho)lipid delivery composition, in particular a lamellar delivery composition in co-formulation with a polynucleotide and optionally an active substance may be applied to cells in vitro, ex vivo, and in vivo, particularly for transfection of eukaryotic cells or tissues including animal cells, human cells, non-human animal cells, insect cells, plant cells, avian cells, fish cells, mammalian cells, mammalian stem cells and the like. The target cell or cells can then be incubated with the co-formulated (phospho)lipid delivery composition, suitably a lamellar delivery composition, or, for in vivo applications, the co-formulated (phospho)lipid delivery composition and polynucleotide can be administered to the organism so that the (phospho)lipid delivery composition contacts the target cells or tissue. The (phospho)lipid delivery composition, suitably a co-formulated lamellar delivery composition and polynucleotide co-formulation may also be conjugated to or mixed with or used in conjunction with a variety of useful molecules and substances, (delivery helpers), such as proteins, peptides, hydrophilic and lipophilic drugs and the like to enhance cell-targeting, uptake, internalization, nuclear targeting and expression.

Suitably, the co-formulated (phospho)lipid delivery composition, for example a lamellar delivery composition, may be used to generate transfected cells which express useful gene products. Suitably the co-formulated (phospho)lipid delivery composition may be used in methods of gene therapy, for example to introduce polynucleotides selected from a group comprising, for example, antisense nucleic acids, ribozymes, RNA regulatory sequences, siRNA, RNAi, Stealth® RNAi, conjugates of RNA with fatty acids or other fat-like molecules, or related inhibitory or regulatory nucleic acids into cells. In particular, these methods may be useful in cancer treatment, in in vivo and ex vivo gene therapy, and in diagnostic methods.

Suitably the co-formulated (phospho)lipid delivery composition, for example the lamellar delivery composition, may be provided to allow delivery of polynucleotide and optionally an active substance to fibroblast cells, macrophage cells, dendritic cells, lung alveolar cells, epithelial cells, embryonic stem cells, induced pluripotent stem cells or the like. It is understood that particularly the presence of negatively charged lipids in the compositions may stimulate the uptake of the compositions by these cells.

In embodiments the provided co-formulated (phospho)lipid delivery composition and polynucleotide and optionally an active substance may be suitable for local administration to the lung by means of tracheal instillation or inhalation.

In embodiments the provided co-formulated (phospho)lipid delivery composition and polynucleotide and optionally an active substance may be suitable for administration to or into the nose, eye or ear (intranasal, intra-auricular; intra-ocular, respectively)

In embodiments the co-formulated (phospho)lipid delivery composition and polynucleotide and optionally an active substance may be suitable for intra-articular or intracerebral or intrathecal or intra-lumbal or intraperitoneal administration.

In embodiments the co-formulated (phospho)lipid delivery composition and polynucleotide and optionally an active substance may be suitable for parenteral administration, including intradermal, subcutaneous, intramuscular or intravenous administration.

In embodiments the co-formulated (phospho)lipid delivery composition and polynucleotide and optionally an active substance may be suitable for administration to the intact or damaged or diseased skin. For example, the co-formulated (phospho)lipid may be provided as a topical, including dermal formulation.

In embodiments the co-formulated (phospho)lipid delivery composition and polynucleotide and optionally an active substance may be suitable for buccal, sublingual, enteral or oral administration.

In embodiments the co-formulated (phospho)lipid delivery composition and polynucleotide and optionally an active substance may be suitable for rectal administration.

In embodiments the co-formulated (phospho)lipid delivery composition and polynucleotide and optionally an active substance may be used followed by the sequential administration of an active substance. Suitably, the co-formulated (phospho)lipid delivery composition and polynucleotide may be provided with an active substance simultaneously, separately, or sequentially.

An additional active substance may be encapsulated or non-encapsulated in the vesicle provided by the (phospho)lipid delivery composition. Suitably the polynucleotide of the co-formulation is not ionically bound to or fully located within the (phospho)lipid vesicle. Suitably the polynucleotide may be shielded from the environment or sheathed or cloaked by vesicles of the (phospho)lipid composition in the co-formulated (phospho)lipid delivery composition.

Preferred features and embodiments of each aspect of the invention are as for each of the other aspects mutatis mutandis unless context demands otherwise.

### Definitions

Each document, reference, patent application or patent cited in this text is expressly incorporated herein in their entirety by reference, which means it should be read and considered by the reader as part of this text. That the document, reference, patent application or patent cited in the text is not repeated in this text is merely for reasons of conciseness.

Reference to cited material or information contained in the text should not be understood as a concession that the material or information was part of the common general knowledge or was known in any country.

Throughout the specification, unless the context demands otherwise, the terms

'Comprise' or 'include', or variations such as 'comprises' or 'comprising', 'includes' or 'including' will be understood to imply the includes of a stated integer or group of integers, but not the exclusion of any other integer or group of integers.

As used herein, the articles "a" and "an" refer to one or to more than one (for example to at least one) of the grammatical object of the article.

"About" shall generally mean an acceptable degree of error of maximally 10 % for the quantity measured given the nature or precision of the measurements.

"Treat", Treating" or "Treatment" refers to therapy, prevention and prophylaxis and particularly refers to the administration of medicine or the performance of medical procedures with respect to a patient, for either prophylaxis (prevention) or to cure or reduce the extent of or likelihood of occurrence of the infirmity or malady or condition or event in the instance where the patient is afflicted.

"Transfection" as used herein means the delivery of nucleic acid, to a target cell, such that the nucleic acid, is expressed, processed or has a biological function in the cell.

The term "active substance" or "active", when used herein can refer to any substance that is to be conveyed to the interior of a cell either in culture in a laboratory or in cells in a tissue in an animal or a human. An active substance may be a macromolecule, a protein, or a peptide, or may be a drug or another organic small molecule. Suitably, the active substance may furnish pharmacological activity or other direct effect in the diagnosis, cure, mitigation, treatment or prevention of disease or to affect the structure and function of the body.

"Drug" or "drug substance", also described as "active" or "active substance" refers to any therapeutic or prophylactic agent other than food or feed which is used in the prevention, diagnosis, alleviation, treatment, or cure of disease in man or animal.

A polynucleotide can be a nucleic acid such as, e.g., deoxyribonucleic acid (DNA),ribonucleic acid (RNA). In some embodiments, the polynucleotide can be a DNA molecule. The DNA can be either linear DNA or circular DNA, such as DNA in the form of a circular plasmid, an episome or an expression vector. In embodiments, polynucleotide can be complementary DNA (cDNA) having an expressible nucleic acid sequence, including at least one open reading frame operably linked to one or more nucleic acid sequence required for the transcription of an mRNA from the expressible nucleic acid sequence. In embodiments, polynucleotide can be an RNA molecule. The RNA molecule may be any type of RNA molecule, without limitation, including but not limited to an mRNA, an siRNA, a miRNA, an antisense RNA, a ribozyme, or any other type or species of RNA molecule familiar to those skilled in the art without limitation, that would be sought to be delivered to the interior of a cell.

The polynucleotide e.g. DNA or RNA may encode an expressible nucleic acid sequence, such as an expression vector or a cDNA molecule comprising an open reading frame encoding a protein. Other suitable molecules that may function as suitable active substances include RNA molecules, such as an mRNA molecule or an RNAi molecule or microRNA (miR). Suitably delivery of a nucleic acid by a lamellar (phospho)lipid delivery composition provides for transfection of nucleic acids into a cell.

Suitably, an active substance provided with a nucleotide may be a macromolecule, a transfection agent or drug. Suitably the active substance may be provided in association with the lamellar delivery composition and simultaneously non complexed with the lamellar delivery composition.

Suitably a polynucleotide may be DNA, RNA, PNA, miRNA, locked nucleic acids ribozymes, peptides, proteins, SiRNA, RNAi, aptamers or spiegelmers. Suitably the nucleic acid may be a functional nucleic acid that can exert an effect on a cell, for example RNAi, siRNA, antisense nucleic acid, a ribozyme, aptamer, and spiegelmer or other gene regulating or gene editing nucleic acid, in particular embodiments the nucleic acid can be DNA.

Suitably an active substance may comprise drug substances selected of the group consisting of antivirals, anti-parasitic, antibiotics, immuno-modulators / immune-suppressants, anti-inflammatories (anti-phlogistics) and cytostatics.

### Dosage forms

Co-formulations of (phospho)lipid delivery composition and polynucleotide to be delivered can be provided in a variety of pharmaceutical compositions and dosage forms for therapeutic applications. For example, they can be incorporated in injectable formulations, intranasal formulations and formulations for intravenous and/or nasal or pulmonary administration.

In case liquid formulations comprising an aqueous phase are being used, the pH should be adjusted to the pH which is physiologically compatible at which there is a minimum of hydrolysis of the phospholipids. In addition, anti-oxidants may be added to suppress the oxidation of lipids containing unsaturated fatty acids. In that case also protective gases like nitrogen or argon may be used.

For pulmonary administration, dosage forms suitable for tracheal instillation comprise liquid formulations which are sterile, preferably endotoxin free, isotonic and iso-hydric. The liquid formulations are preferable aqueous formulations. They may contain low percentages of organic solvents like polyethylene (PEG) 300 or 400, propylene glycol and ethanol. In some cases also hypertonic salt solutions may be used to enhance the mucus diluting effect of the (phospho)lipid compositions.

For inhalation purposes the compositions may be dispersed in aqueous solutions suitable for nebulization or formulated in dry powders for inhalation. In both cases, the powder or liquid to be inhaled should have an appropriate particle size, allowing sufficient lung disposition.

For injections the dosage form should be sterile, endotoxin-free, preferably isotonic and isohydric. Isotonizing agents are e.g. NaCl and glucose etc, which are known in the art. The (phospho)lipid delivery composition should preferably comprise particles with a size smaller than 200 nm, allowing sterile filtration.

The aqueous formulations may contain mono- or di-saccharides or other suitable cryo- or lyo-protectants when the formulations need to be stabilized by means of freezing or freeze-drying.

For administration to the skin, the aqueous dispersion of the co-formulation of (phospho)lipid delivery composition and polynucleotide, optionally further comprising an active substance, may be formulated in a gel, so maintaining the particle size of the lipid particles.

Dosage forms of the (phospho)lipid delivery composition and polynucleotide co-formulation comprising so called pro-liposomes may be of particular interest for parenteral and topical administration. Liquid pro-liposomes are concentrates of the lipids in pharmaceutically acceptable organic solvents, like ethanol, glycerol and propylene glycol, which upon (in- situ) dilution with an aqueous medium result in an aqueous dispersion of the lamellar structures. The final dispersion contains the organic solvent at acceptable pharmaceutical, non-toxic level. Dry pro-liposome comprise a lyophilisate of the lipid components, which upon (in-situ) hydration results in an aqueous dispersion of the lamellar structures. Hydrophilic, water soluble drugs can be integrated in dry and liquid liposomes by dissolving the drug in the aqueous phase used to prepare the delivery co-formulations. Lipophilic drugs can be integrated in dry and liquid liposomes by dissolving the drug in the organic phase uses to dissolve the lipids (liquid pro-liposomes) or in the organic phase used to prepare the lyophilisation of the lipid components.

### Kit

Suitably the (phospho)lipid delivery compositions, for example lamellar delivery compositions and polynucleotide may be provided as a kit. The kit may contain a polynucleotide to be transferred to the intracellular part of the cell by using a (phospho)lipid delivery composition, for example a lamellar delivery composition, which is *in-situ* formed. This kit can also optionally include a transfection enhancing agent such as a transfection enhancing peptide, protein or fragment thereof or a transfection enhancing compound mixture (i.e. in a single container, typically a tube and/or vial), or can be included as separate portions (i.e. in separate containers, for example separate vials and/or tubes). The kits of the present invention, as will be understood, typically include vessels, such as vials and/or tubes, which are pack- aged together, for example in a cardboard box or other pack- aging. The kit can also include in separate containers, cells, cell culture medium, and a reporter nucleic acid sequence, such as a plasmid that expresses a reporter gene.

In further aspects, the kit may comprise dry or liquid pro-liposomes. Suitably, the combination of liquid pro liposomal concentrate in one vial and the aqueous solution of the polynucleotide and optionally, for example, a drug in another vial may comprise a kit for in-situ use. Suitably the liquid pro liposomal concentrate in one vial may contain a lipophilic drug. Suitably the combination of lipid components and lipophilic drug in a lyophilisate in one vial and another vial with an aqueous medium may comprise a kit for in-situ use. Suitably, the combination of lipid components in a lyophilisate in one vial and another vial with an aqueous medium containing a drug may comprise a kit for in situ use. Suitably, the (phospho)lipid delivery composition co-formulation may be prepared with a hydrophilic or lipophilic drug and used as a kit as such or after lyophilisation with suitable cryo-protectants in one vial and hydration medium, optionally containing a hydrophilic drug, in another vial.

### Manufacturing methods

Suitably (phospho)lipid drug delivery composition may be formed by dispersing the mixed lipids in aqueous media, and may be produced using techniques such as extrusion, freeze-thaw or sonication.

Production of the phospholipid delivery composition can comprise preparation of a lipid film by rota-evaporation of an organic phase containing the lipids, optionally with a lipophilic drug, followed by hydration of the dry lipid film with an aqueous phase, optionally containing a water soluble drug. The resulting lipid dispersion, when prepared without the polynucleotide, can be mixed with an aqueous polynucleotide solution. Also the lipid dispersion may be extruded though filters or high shear homogenization or sonication can be applied to down size the vesicle size.

At larger scale, the lipid blend, optionally containing a lipophilic drug, may be prepared by means of drying technologies, like spray drying or freeze drying, to remove the water immiscible or water miscible solvent, followed by hydration of the dry blend, with an aqueous medium, optionally followed by size reduction of the dispersed lipid particles by e.g. extrusion or high pressure homogenization, followed, optionally ,by removal of the unentrapped water soluble drug by methods like cross flow filtration. The final aqueous dispersion of the delivery co-formulation may be used as such or, optionally freeze dried in case one of the components is not suitable for storage in the liquid form.

The dry lipid blend obtained after removal of the solvent by lyophilisation may be used as dry pro-liposomal form as described above. Suitable solvents are e.g. tert-butanol, mixtures of tert-butanol and water and cyclohexane or mixtures thereof. Optionally, glacial acetic acid may be used. These lyophilisates are particularly suitable for preparation of larger liposomes (> 0.2 µm diameter)

Alternatively, in case of using a water miscible solvent the (phospho)lipid delivery compositions may be prepared either by controlled dilution with water or use as such as liquid pro liposomal concentrate for in situ use. The added aqueous phase may optionally contain a hydrophilic drug. The un-encapsulated fraction of the water soluble drug may be removed by cross-filtration techniques.

Suitably, the (phospho)lipid composition may be provided as a lyophilised powder, for example stored at -20 °C. The (phospho)lipid composition may be reconstituted, for example to an initial concentration of 20 mg/ml by adding 5 ml sterile 0.9 % w/v sodium chloride. The saline may have a pH in the range of 7-8. The (phospho)lipid composition may be further diluted, for example to provide a working concentration of 2 mg/ml in saline.

Suitably, for parenteral use or for inhalation the final dispersion or intermediate stages may be sterilized by sterile filtration through 0.2 µm pore size filters. Optionally, the final aqueous dispersion of the (phospho)lipid delivery composition may be sterilized by autoclaving or other techniques known in the art.

Embodiments of the present invention will now be described with reference to the accompanying figures in which:
**Figure 1****:** Overexpression of miR-155 by transfection of Human Pulmonary Fibroblasts using a range of cell number, concentration of (phospho)lipid delivery composition (delivery composition indicated as LMS). Additionally, the expression levels of TGF-β were ascertained by ELISA as a measure of biological activity of the miRNA:LMS combination.
**Figure 2****:** Overexpression of miR-155 by transfection of Human Pulmonary Fibroblasts. HPF were seeded and transfected with Control Mimic (CM) or mimic miR-155 using DharmaFECT 3 (Dharma) as a positive control, or co-formulated (phospho)lipid delivery composition and miRNA. Expression of the miRNA was quantified by qRT-PCR and normalised to untransfected cells.

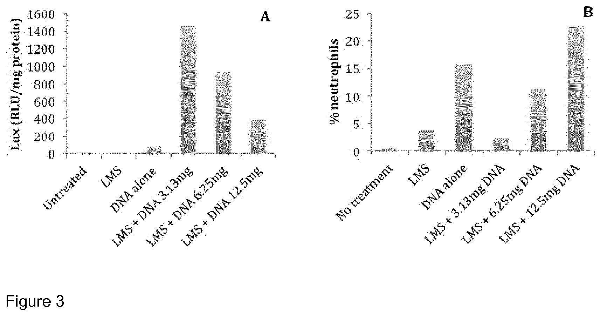

**Figure 3****:** Transfection of the sheep lung by instillation. Sheep received co-formulated (phospho)lipid delivery composition and a plasmid containing the luciferase gene. 24 hours post instillation the sheep lung was processed and analysed for luciferase activity measured by relative light units (RLU) normalised to untransfected tissue (A). Immune cell content of the lung post plasmid delivery. Cells are collected by bronchioalveolar lavage (BAL) and quantified. Neutrophils (an indicator of inflammatory response) were quantified. LMS-611 (trialed (phospho)lipid delivery composition) high is with 12.5 mg of DNA plasmid containing luciferase, LMS-611 (trialed (phospho)lipid delivery composition) Mid has 6.25 mg of plasmid and LMS-611 (trialed (phospho)lipid delivery composition) low has 3.13 mg of plasmid (B).

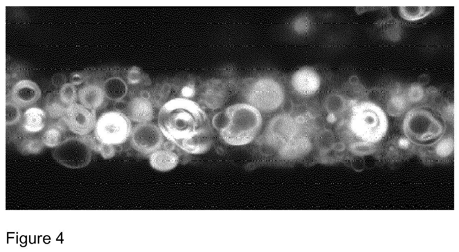

**Figure 4****:** Photograph of (phospho)lipid composition sheathing nucleic acid to provide co-formulated (phospho)lipid delivery composition.

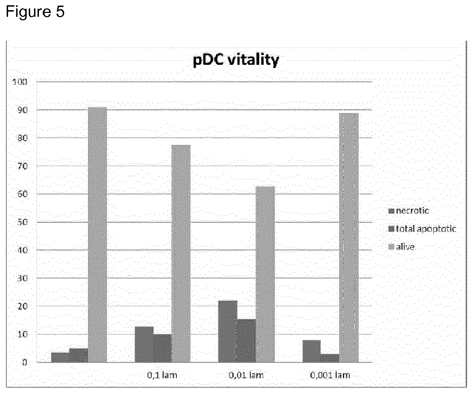

**Figure 5****:** Change in cell vitality following provision of co-formulated (phospho)lipid delivery composition and active to a cell.
**Figure 6****:** Transfection of M2 macrophages with co-formulated (phospho)lipid delivery composition comprising and active substance of a nucleic acid to induce downregulation of CCL18.
**Figure 7****:** Transfection of fluorescently labelled nucleic acids into M2 macrophages using lamellar bodies.
**Figure 8****:** Transfection of human pulmonary fibroblasts with fluorescent miR-155 mimic molecules using co-formulated (phospho)lipid delivery composition and subsequent change in cellular concentration of miR-155 and TGF-β.

### Examples

### Example 1

A (phospho)lipid composition comprising 54% phosphatidylcholine, about 19% sphingomyelin, about 8% phosphatidylethanolamine, about 4% phosphatidylserine, about 3% phosphatidylglycerol and about 10% cholesterol by weight was provided as a lyophilised powder in quantities of 100 mg. Before use, phospholipid composition in this form was stored at -20 °C. Under sterile conditions, the phospholipid composition was reconstituted to an initial concentration of 20 mg/ml by adding 5 ml sterile 0.9 % w/v sodium chloride directly to the composition using a needle and syringe. The saline used had a pH of 7-8. For use in transfections, this initial concentration of phospholipid composition was diluted to a working concentration of 2 mg/ml by taking 100 µl phospholipid composition and adding it to a sterile Eppendorf tube containing 900 µl 0.9 % w/v sodium chloride. This working concentration was then diluted as required, depending on the experiment.

### Transfection protocol for delivery of miRNA mimic to cells in vitro

Preparation of all cell transfection reagents was carried out under sterile conditions. Cells were prepared for transfection by removing the cell culture medium in which they were cultured and replacing it with 500 µl fresh media. (Phospho)lipid composition, reconstituted to a working concentration of 2 mg/ml as described above, was used to prepare a transfection reaction mix containing (phospho)lipid composition, miRNA mimic and saline, by mixing the pre-prepared (phospho)lipid composition with an aqueous solution of the miRNA. The reaction mix was mixed thoroughly and incubated at room temperature for 20-30 minutes to provide a co-formulated phospholipid delivery composition and miRNA, in which it is understood the miRNA is non-encapsulated in the (phospho)lipid delivery composition. 15 µl amounts of this co-formulated phospholipid delivery composition and miRNA mimic was added slowly, drop-by-drop in different areas of a well, and a plate containing the well was shaken gently for 2 seconds to mix the contents of the wells. miRNA was used at a concentration of 50nM in (phospho)lipid delivery composition and tested against commercial transfection reagents.

The co-formulated phospholipid delivery composition and miRNA was most efficacious with miR-155 at a concentration of 10 µg/mL with the miRNA used at 50 nM.

For use with the pCIK Lux plasmid in a Roslin sheep model measuring luciferase expression, the phospholipid delivery composition concentration used was 9.8 mg/mL relative to 3.125mg, 6.25mg and 12.5 mg of plasmid .

For use with the dendritic cells, the phospholipid composition was used at a range between 0.01 and 1 mg/mL

### Example 2

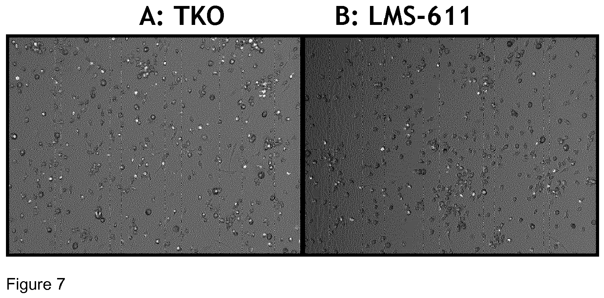

A co-formulated (phospho)lipid delivery composition was prepared, to test the transfection of a nucleic acid into M2 macrophages. Co-formulated (phospho)lipid delivery composition with nucleic acid for delivery into a cell were prepared, as described in Example 1, where the nucleic acid was fluorescently labelled and the M2 macrophages were seeded and transfected using TKO transfection reagent as a positive control. A microscope image of such is shown in Figure 7.

To test the way in which cells were affected by co-formulated (phospho)lipid delivery composition and nucleic acid active substance, plasmacytoid dendritic cells (pDC) were treated with the co-formulated (phospho)lipid delivery composition at a range of concentrations. The cells were then tested using highly fluorescent Annexin V (AnnV) binding to assess the presentation of 7-amino-actinomycin D (7-AAD).

7-AAD has a high DNA binding constant and is efficiently excluded by intact cells. It is useful for DNA analysis and dead cell discrimination during flow cytometric analysis. AnnV conjugates provide quick and reliable detection methods for studying the externalization of phosphatidylserine. In normal viable cells, phosphatidylserine (PS) is located on the cytoplasmic surface of the cell membrane. However, in the intermediate stages of apoptosis, PS is translocated from the inner to the outer leaflet of the membrane, exposing PS to the external cellular environment where it can be detected. As shown in Figure 3, the addition of the co-formulated (phospho)lipid composition and nucleic acid active at various concentrations has no significant effect on cell vitality.

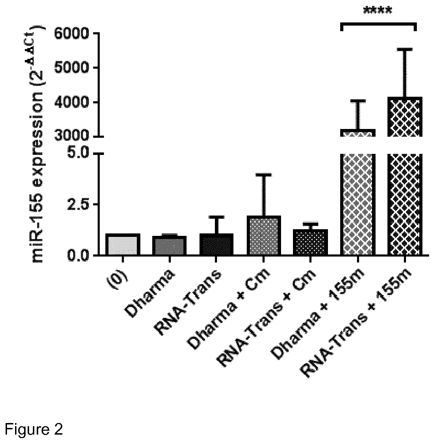

To demonstrate upregulation of gene expression, transfection with the co-formulated (phospho)lipid delivery composition capable of providing for overexpression of miR-155 in human pulmonary fibroblasts (HPF) was undertaken. The co-formulated (phospho)lipid delivery composition with nucleic acids encoding Control Mimic (CM) or mimic miR-155 were prepared. HPF were seeded and transfected with Control Mimic (CM) co-formulated (phospho)lipid delivery composition or mimic miR-155 co-formulated (phospho)lipid delivery composition using DharmaFECT 3 (Dharma) as a positive control, containing cationic amino-lipids, and blank co-formulated (phospho)lipid delivery composition (RNA-Trans) as a negative control. Expression of miR-155 was quantified by qRT-PCR and normalised to untransfected cells, as shown in Figure 2.

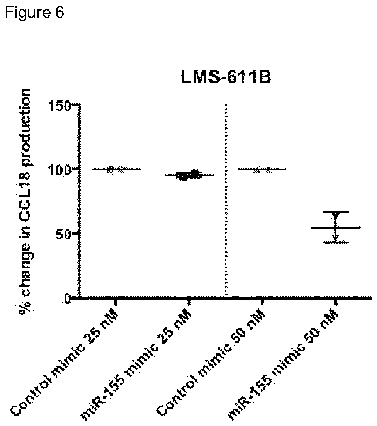

To test the downregulation of CCL18 in M2 macrophages, co-formulated (phospho)lipid delivery composition with nucleic acids was prepared, encoding mimic miR-155. M2 macrophages were seeded and mimic miR-155 co-formulated (phospho)lipid delivery composition transfected at two concentrations. A dose-response by way of a decrease in functional CCL18 after miR-155 transfection using co-formulated phospholipid delivery composition was measured by ELISA, as shown in Figure 6.

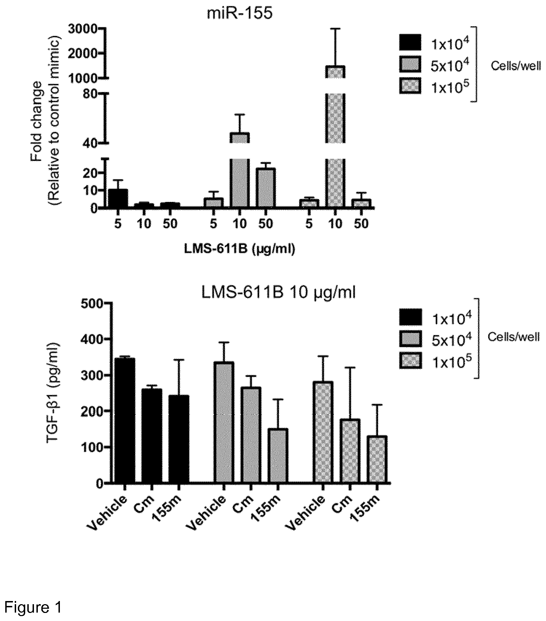

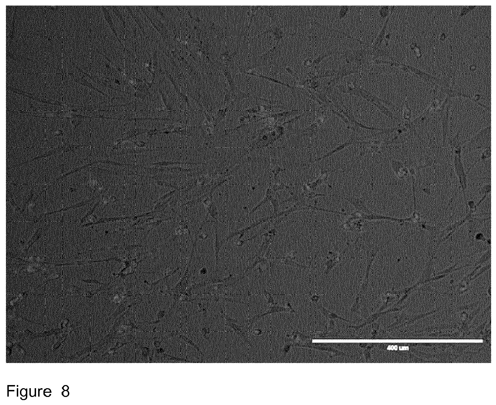

As a second example of gene expression downregulation, following transfection using co-formulated (phospho)lipid delivery composition with fluorescently labelled miR-155 was used to produce co-formulated (phospho)lipid delivery composition at a range of miR-155 concentrations and these were used to transfect human pulmonary fibroblasts (HPF). The cells were imaged using light microscopy (Figure 8) to illustrate localisation of the fluorescently labelled mimic miR-155. An experiment was then conducted comparing the induced increased cellular concentration of miR-155 with the downstream downregulation of TGF-β using the above co-formulated (phospho)lipid delivery composition fluorescently labelled mimic miR-155 co-formulated (phospho)lipid delivery composition and negative control co-formulated (phospho)lipid delivery composition control mimic (Cm). HPF cells were seeded, transfected and quantified by real time quantitative PCR, as shown in Figure 1.

Further, co-formulated (phospho)lipid delivery composition with a plasmid to be delivered containing the luciferase gene at amounts of 3.125mg, 6.25mg and 12.5 mg were prepared for administration by inhalation and administered to sheep by instillation. At 24 hours post instillation the sheep lung was processed and analysed as shown in Figure 3.

Luciferase activity was measured by relative light units (RLU) normalised to untransfected tissue, and the immune cell content of the lung post (phospho)lipid delivery was considered. Cells are collected by bronchioalveolar lavage (BAL) and quantified. Neutrophils (an indicator of inflammatory response) were quantified. LMS-611 (trialed (phospho)lipid delivery compsition) high is a co-formulated (phospho)lipid delivery composition with 12.5 mg of DNA plasmid containing luciferase, LMS-611 Mid is a co-formulated (phospho)lipid delivery composition with 6.25 mg of plasmid and LMS-611 low is a co-formulated (phosphor)lipid delivery composition of 3.13 mg of plasmid (B).

Although the invention has been particularly shown and described with reference to particular examples, it will be understood by those skilled in the art that various changes in the form and details may be made therein without departing from the scope of the present invention.

## Claims

1. A co-formulated (phospho)lipid delivery composition and polynucleotide to allow delivery of the polynucleotide into a cell wherein the (phospho)lipid delivery composition does not contain any one or a combination of amino lipids, pegylated lipids, and divalent ions.

2. A co-formulated (phospho)lipid delivery composition and polynucleotide to allow delivery of the polynucleotide to a cell wherein the (phospho)lipid delivery composition, comprises at least one of the lipids selected from phosphatidylcholine (PC), phosphatidylglycerol (PG), sphingomyelin (SM), phosphatidylethanolamine (PE), phosphatidylserine (PS), phosphatidylinositol (PI), phosphatidic acid (PA), and optionally cholesterol wherein the (phospho)lipid delivery composition further comprises phospholipids, which individually, are negatively charged at pH 7 and are optionally selected from at least one of PI, PS, PG and PA.

3. The co-formulated (phospho)lipid delivery composition as claimed in any one of claims 1 or 2 wherein polynucleotide is not encapsulated inside a vesicle of the (phospho)lipid delivery composition.

4. The co-formulated (phospho)lipid delivery composition as claimed in any one of claims 1 to 3 wherein the (phospho)lipid delivery composition comprises, phosphatidylcholine and a negatively charged lipid at pH7.

5. The co-formulated (phospho)lipid delivery composition as claimed in any one of claims 1 to 4 comprising a weight ratio of polynucleotide to total lipid from 1:1000 to 1:1 to a weight ratio of polynucleotide to total lipid from 1000:1 to 1.1.

6. The co-formulated (phospho)lipid delivery composition as claimed in any one of claims 1 to 5 comprising at least one phospholipid selected from phosphatidylcholine (PC), phosphatidylglycerol (PG), sphingomyelin (SM), phosphatidylethanolamine (PE), phosphatidylserine (PS), phosphatidylinositol (PI), phosphatidic acid (PA) or salts thereof in combination with cholesterol.

7. The co-formulated (phospho)lipid delivery composition as claimed in any one of claims 1 to 6 comprising a combination of cholesterol, sphingomyelin (SM), phosphatidylcholine (PC), and at least one of phosphatidic acid (PA), phosphatidylglycerol (PG), phosphatidylinositol (PI) and phosphatidylserine (PS).

8. The co-formulated (phospho)lipid delivery composition as claimed in any one of claims 1 to 6 comprising a combination of cholesterol, sphingomyelin (SM), phosphatidylcholine (PC), and at least one of phosphatidylserine (PS) and phosphatidylglycerol (PG).

9. The co-formulated (phospho)lipid delivery composition as claimed in any one of claims 1 to 8 wherein the measured average particle size for the (phospho)lipid delivery composition is in the range (a) average size from 20nm to 200 nm diameter, or (b) average size from greater or equal to 200nm to 80 microns in diameter.

10. The co-formulated (phospho)lipid delivery composition as claimed in any one of claims 1 to 9 wherein vesicles provided by the (phospho)lipid delivery composition have a zeta potential in the range -4 mV to about -70 mV.

11. The co-formulated (phospho)lipid delivery composition as claimed in any one of claims 1 to 10 further comprising an additional active substance.

12. A co-formulated (phospho)lipid delivery composition and polynucleotide of any one of claims 1 to 11 for use in the treatment of a condition or disease wherein a cell to which the polynucleotide is to be delivered is covered in at least a layer of at least one of mucus, bacterially derived alginate and biofilm.

13. A co-formulated (phospho)lipid delivery composition and polynucleotide of any one of claims 1 to 12 for use in the treatment of a condition or disease **characterised by** alteration of mucous secretions, optionally wherein the condition is selected from COPD, cystic fibrosis, idiopathic pulmonary fibrosis, fibrotic lung diseases, and genetic disorders.

14. A method of preparing a co-formulated (phospho)lipid delivery composition and polynucleotide to allow intracellular delivery of the polynucleotide wherein the (phospho)lipid delivery composition does not contain any one or a combination of amino lipids, pegylated lipids and divalent ions and the method comprises
a) providing a simultaneous mixture of delivery composition and polynucleotide, or,
b)
i) providing sequentially, a separate delivery composition and a separate solution of the polynucleotide which can be provided in combination to a subject or a cell to provide a co-formulated delivery composition without a further active substance or
ii) providing sequentially, as a separate delivery composition and a separate solution of the polynucleotide which can be provided in combination to a subject or a cell to provide a co-formulated delivery composition with a further encapsulated/associated active substance wherein the active substance is not an independent interaction moiety.

15. Use of the co-formulated (phospho)lipid delivery composition and polynucleotide of any one of claims 1 to 11 in the ex-vivo manipulation of cells isolated from a body, optionally to allow delivery of polynucleotide to dendritic cells to modulate immune response.
